# EUROPÄISCHE PATENTANMELDUNG

(11) **EP 1 010 412 A2**
(43) Veröffentlichungstag der Anmeldung: **21.06.2000**
(21) Anmeldenummer: 99124784.2
(22) Anmeldetag: 14.12.1999
(51) Int. Cl.: A61J 1/00

(54) **Adapter für enterale Überleitgeräte**

(30) Priorität: 17.12.1998 DE 19858237
(71) Anmelder: Fresenius Kabi Deutschland GmbH, 61352 Bad Homburg v.d.H. (DE)
(72) Erfinder: Knierbein, Bernd, Dr., 61267 Neu-Anspach (DE)
(74) Vertreter: Luderschmidt, Schüler & Partner GbR

(57) **Zusammenfassung**

Ein Adapter zum Anschluß von enteralen Überleitgeräten an Flüssigkeit enthaltende Behältnisse verfügt über ein als Schraubverschluß ausgebildetes Basisteil (1), das einen Durchgang aufweist. An das Basisteil schließt sich ein rohrförmiges Aufnahmestück (5) zum Einführen eines Spike (6) an, der an dem Überleitgerät vorgesehen ist. Der Durchgang (7) des Basisteils ist mit einer geschlitzten Ventilscheibe (8) aus flexiblen Material verschlossen, die sich beim Einführen des Spike in das rohrförmige Aufnahmestück (5) zur Herstellung einer Flüssigkeitsverbindung öffnet. Der Adapter ermöglicht den Anschluß der bekannten Überleitgeräte an handelsübliche Glas- oder Kunststofflaschen mit Standardschraubverschluß. Separate Beutel, die vom Patienten mit Flüssigkeit gefüllt werden, sind zur Bilanzierung somit nicht erforderlich.

## Beschreibung

Die Erfindung betrifft einen Adapter zum Anschluß von enteralen Überleitgeräten an Flüssigkeiten enthaltende Behältnisse.

Die bekannten Nährlösungen zur enteralen Ernährung werden in Flaschen oder Beuteln bereitgestellt. Die Überführung der Nährlösung erfolgt mit einem Überleitgerät, das einerseits an den die Nährlösung enthaltenden Behälter und andererseits an die in den Magen des Patienten führende Ernährungssonde angeschlossen wird. Zur Konnektierung des Überleitgeräts weist der Behälter einen Anschlußteil auf, der mit dem komplementären Konnektor des Überleitgeräts verbunden wird.

Aus der DE 196 17 024 A1 ist ein enteraler Nährlösungsbeutel bekannt, der über einen Port zum Anschluß eines Spikes verfügt. Der Port ist mit einer Membran verschlossen, die beim Anschluß der Überleitgerätes mit dem Spike durchstochen wird.

Neben der Nährlösung muß dem Patienten eine ausreichende Menge an Flüssigkeit zugeführt werden. Hierzu sind Bilanzierbeutel bekannt, die vom Patienten selbst mit Mineralwasser oder anderen Flüssigkeiten befüllt werden können. Die Bilanzierbeutel verfügen über Anschlußteile, die sich mit den bekannten Überleitgeräten verbinden lassen. Der Anschluß von handelsüblichen Industriegetränkeflaschen mit Standardschraubverschluß an die bekannten Überleitgeräte ist hingegen nicht möglich. Daher kann die Flüssigkeitsbilanzierung nur mit den Bilanzierbeuteln vorgenommen werden, verbunden ist. Darüber hinaus besteht beim Umfüllen der Getränke aus den handelsüblichen Flaschen in den Bilanzierbeutel eine zusätzliche Verkeimungsgefahr.

Die DE 38 28 729 C2 beschreibt einen Adapter zum Anschluß eines enteralen Überleitgerätes an Kronkorkenverschlüsse unterschiedlicher Größe. Der Adapter weist zwei Kappen mit verschiedenen Öffnungsdurchmessern für die unterschiedlichen Kronkorkenverschlüsse auf. Die innere Kappe ist mit einem Durchlaß für die Nährlösung und einem Entlüftungsventil versehen. Der Anschluß von Industriegetränkeflaschen mit Standardscbraubverschluß an die bekannten Überleitgeräte ist auch mit diesem Adapter nicht möglich.

Aus der DE 87 03 587 U 1 ist ein Adapter zum Anschließen von enteralen Überleitungsgeräten anFlaschen mit Schraubverschluß, insbesondere an Babyflaschen bekannt, der aus einem Stutzen und einer Überwurfmutter besteht. Zur Flüssigkeitsbilanzierung ist der bekannten Adapter nicht bestimmt.

Der Erfindung liegt die Aufgabe zugrunde, einen Adapter zum Anschluß von enteralen Überleitgeräten an Behältnisse mit Standardschraubverschluß zu schaffen, der die Flüssigkeitsbilanzierung des Patienten vereinfacht.

Die Lösung dieser Aufgabe erfolgt erfindungsgemäß mit den im Patentanspruch 1 angegebenen Merkmalen.

Der erfindungsgemäße Adapter verfügt über ein Basisteil, das als Schraubkappe ausgebildet ist, die auf handelsübliche Getränkebehältnisse, beispielsweise Glas-oder Kunststofflaschen mit Standardschraubverschluß, aufgeschraubt werden kann. Zur Flüssigkeitsbilanzierung braucht der Patient das Überleitgerät nur von dem enteralen Nährlösungsbehälter abzunehmen und das Überleitgerät an die Getränkeflasche anzuschließen. Mit der Flüssigkeit aus der Flasche kann das Überleitgerät auch jederzeit freigespült werden. Das Befüllen eines separaten Bilanzierbeutels erübrigt sich somit.

Der Durchgang des Basisteils für die dem Patienten zuzuführende Flüssigkeit wird von einer geschlitzten Scheibe aus flexiblem Material verschlossen. Das Scheibenventil öffnet sich beim Einführen des Spike in das rohrförmige Aufnahmestück des Adapters, so daß die Flüssigkeitsverbindung automatisch hergestellt wird. Da die Flüssigkeitsverbindung beim Abnehmen des Überleitgerätes sofort unterbrochen wird, kann das Überleitgerät wechselweise auch dann an unterschiedliche Behältnisse angeschlossen werden, wenn diese über Kopf aufgehängt sind, was die Handhabung der Behälter bzw. des Überleitgerätes weiter vereinfacht. Der sich nach Abnehmen des Überleitgerätes selbsttätig verschließende Adapter erlaubt dem Patienten nicht nur einen Teil der Flüssigkeitsmenge zu geben, sondern auch nach Verabreichung der Nährlösung, das Überleitgerät zu spülen.

Die Schraubkappe des Adapters ist zum Anschluß an Getränkebehältnisse mit Standardschraubverschluß bestimmt. Die Gewinde der handelsüblichen Getränkeflaschen unterscheiden sich nur geringfügig in Gewindedurchmesser und Steigung. Die Schraubkappe ist derart ausgelegt, daß sie trotz dieser Toleranzen auf die handelsüblichen Getränkeflaschen aufgeschraubt werden kann. Da aber eine optimale Paßgenauigkeit der Schraubkappe nicht gegeben werden kann, ist eine sichere Abdichtung von großer Bedeutung. Diese wird vorteilhafterweise durch ein in die Schraubkappe eingesetztes Dichtelement erreicht, das gegenüber dem Flaschenrand abdichtet. Die ansonsten bei Glas- oder Kunststofflaschen üblichen Dichtlippen, die an der Innenkante der Flaschen abdichten, haben sich wegen der unterschiedlichen Innendurchmesser der Mündungen nicht als brauchbar erwiesen. Um die Gasdichtigkeit zu verbessern, kann an dem Dichtelement noch ein umlaufender Ansatz vorgesehen sein, der gegenüber dem Außenrand des Getränkebehälters abdichtet.

Der Adapter kann als Spritzgießteil vorzugsweise aus Polypropylen oder Polyethylen gefertigt sein. Um den Adapter an nicht kollbabierbare Gebinde, beispielsweise Glas-oder Kunststofflaschen, anschließen zu können, ist an der Schraubkappe ein Entlüftungsventil vorgesehen. Ohne Entlüftungsventil kann der Adapter nur an kollabierbare Gebinde, beispielsweise Beutel oder Faltflaschen, angeschlossen werden, die über Standardschraubverschlüsse verfügen.

Bei einer bevorzugten Ausführungsform des Adapters wird die geschlitzte Scheibe klemmend in der Schraubkappe von einem Einsatzteil gehalten, das wiederum in der Schraubkappe einrastend fixiert ist. Dadurch wird die Herstellung des Adapters als Spritzgießteil vereinfacht.

Die Verkeimungsgefahr kann mit einer hydrophoben, d.h. für Flüssigkeit undurchlässigen, aber für Gas durchlässigen Membran verringert werden, die den Belüftungskanal in der Schraubkappe verschließt.

Weitere bevorzugte Ausführungsformen der Erfindung sind Gegenstand der Unteransprüche.

Im folgenden wird ein Ausführungsbeispiel des erfindungsgemäßen Adapters unter Bezugnahme auf die Zeichnungen näher erläutert.

Es zeigen:

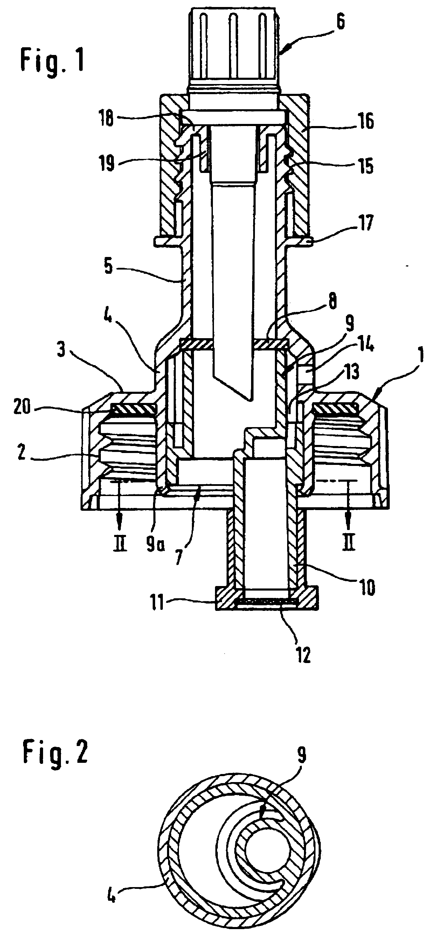

- Figur 1: den Adapter zusammen mit dem Spike eines Überleitgerätes in geschnittener Darstellung und
- Figur 2: einen Schnitt durch den Adapter entlang der Linien II-II von Figur 1.

Der Adapter weist ein als Schraubkappe ausgebildetes Basisteil 1 aus PP oder PE auf. Das Innengewinde 2 der Schraubkappe hat einen Durchmesser von 28 mm und eine Steigung von 3,25 mm, so daß sich der Adapter auf handelsübliche Ein- oder Mehrwegglas- oder -kunststofflaschen aufschrauben läßt, die über einen Standardschraubverschluß verfügen.

Der Deckelteil 3 der Schraubkappe 1 weist eine zentrale Öffnung auf, in der ein zylindrisches Durchgangsstück 4 sitzt, das mit der Schraubkappe einstückig ist. Das sich an seinem oberen Ende verjüngende Durchgangsstück 4 geht in ein rohrförmiges Ansatzstück 5 zur Aufnahme des Spike 6 des Überleitgerätes über. Der Durchgang 7 in der Schraubkappe 1 ist von einer geschlitzten Scheibe 8 aus flexiblem Material, z.B. Silikon, flüssigkeitsdicht verschlossen, die in das Durchgangsstück 4 eingesetzt ist. Derartige Ventilscheiben sind bekannt, so daß sich eine weitere Beschreibung derselben erübrigt. Die sich an der nach innen laufenden Wand des Durchgangsstücks 4 abstützende Ventilscheibe 8 wird von einem Einsatzteil 9 klemmend gehalten, das von unten in das Durchgangsstück eingesetzt und von einer nach innen vorspringenden Nase 9a am unteren Ende des Durchgangsstücks einschnappend fixiert ist.

Das Einsatzteil 9 weist einen rohrförmigen Ansatz 10 auf, der sich seitlich aus der Schraubkappe 1 nach unten erstreckt. Auf dem Ansatz 10 des Einsatzsteils 9 sitzt eine zylindrische Kappe 11, die mit einer hydrophoben, d.h. für Gas durchlässigen, aber für Flüssigkeit undurchlässige Membran 12 verschlossen ist. Das obere Ende des Einsatzteils 9 begrenzt zusammen mit dem Durchgang einen schmalen Spalt 13, der über eine Ausnehmung 14 in dem Durchgangstück 4 seitlich geöffnet ist.

Der rohrförmige Ansatz 5, der sich an das Durchgangsstück 4 anschließt, ist mit einem Außengewinde 15 zum Aufschrauben der Überwurfmutter 16 versehen, mit der sich der Spike 6 des Überleitgeräts mit dem Adapter verschrauben läßt. Ein umlaufender Rand 17 an dem rohrförmigen Ansatz 5 unterhalb des Schraubgewindes 15 bildet einen Anschlag für die Schraubkappe 16.

An seinem oberen Rand weist der rohrförmige Ansatz 4 einen umlaufenden, nach innen vorspringenden Ansatz 18 auf, an den ein Aufnahmestück 19 zur verdrehsicheren Aufnahme des Spike 6 angesetzt ist. Das Aufnahmestück 19 weist einen rechteckförmigen Querschnitt mit abgerundeten Ecken auf, der dem Querschnitt des Spike entspricht.

Zur Abdichtung ist in dem Deckelteil der Schraubkappe 1 ein gegenüber dem Flaschenrand abdichtender Dichtring 20 aus weichem PE, EVA oder Silikon eingesetzt. Die Montage des Dichtelementes kann bei einer gemeinsamen Spritzgießherstellung von Grundkörper und Dichtung aber entfallen.

Zum Anschluß des Überleitgeräts wird der Adapter auf den Schraubverschluß des Getränkebehälters, beispielsweise eine handelsübliche Glas- oder Kunststofflasche aufgeschraubt. Die Konnektion mit dem Spike kann im Hängen, Stehen oder Liegen erfolgen. Mit dem Adapter ist die Flasche flüssigkeitsdicht verschlossen. Daraufhin wird der Spike 6 des Überleitgeräts in den Adapter eingeführt und mit der Überwurfmutter 16 verschraubt. Der Spike 6 durchsticht dabei das Ventilelement 8 des Adapters, so daß die Flüssigkeitsbilanzierung erfolgen kann.

## Patentansprüche

1. Adapter zum Anschluß von enteralen Überleitgeräten an Flüssigkeiten enthaltende Behältnisse mit einem Basisteil (1), das einen Durchgang (7) ausweist und als Schraubkappe ausgebildet ist, die auf eine Flasche aufschraubbar ist, dadurch ge kennzeichnet, daß sich an das Basisteil (1) ein rohrförmiges Aufnahmestück (5) zur Führung eines Spike anschließt, der an dem Überleitgerät vorgesehen ist, wobei der Durchgang (7) des Basisteils mit einer geschlitzten Ventilscheibe (8) aus flexiblem Material verschlossen ist, die sich beim Einführen des Spike (6) in das rohrförmige Aufnahmestück zur Herstellung einer Flüssigkeitsverbindung öffnet, und daß in dem Adapter ein Belüftungsventil (9 bis 13) vorgesehen ist.

2. Adapter nach Anspruch 1, dadurch gekennzeichnet, daß in die Schraubkappe ein gegenüber dem Flaschenrand abdichtendes Dichtelement (20) eingesetzt ist.

3. Adapter nach Anspruch 1 und 2, dadurch gekennzeichnet, daß die geschlitzte Ventilscheibe (8) klemmend in der Schraubkappe (1) von einem Einsatzteil (9) gehalten ist, das in der Schraubkappe einschnappend fixiert ist.

4. Adapter nach Anspruch 3, dadurch gekennzeichnet, daß das Belüftungsventil (9 bis 13) eine hydrophobe Membran (12) aufweist, die einen Belüftungskanal (13) in der Schraubkappe (1) verschließt.

5. Adapter nach Anspruch 4, dadurch gekennzeichnet, daß das Einsatzteil einen aus der Schraubkappe (1) vorspringenden rohrförmigen Ansatz (10) aufweist, der mit der hydrophoben Membran (12) verschlossen ist.

6. Adapter nach einem der Ansprüche 1 bis 5, dadurch gekennzeichnet, daß das rohrförmige Aufnahmestück (5) ein Außengewinde (15) aufweist.

7. Adapter nach einen der Ansprüche 1 bis 6, dadurch gekennzeichnet, daß der Adapter ein Spritzgießteil, vorzugsweise auf Polypropylen oder Polyethylen ist.
